# EUROPÄISCHE PATENTSCHRIFT

(11) **EP 1 074 616 B1**
(45) Veröffentlichungstag und Bekanntmachung des Hinweises auf die Patenterteilung: **20.02.2008**
(21) Anmeldenummer: 00114370.0
(22) Anmeldetag: 05.07.2000
(51) Int. Cl.: C12N 9/64

(54) **Verfahren zur Reindarstellung des Proenzyms der den Blutgerinnungsfaktor VII aktivierenden Protease mittels Ionenaustauscherchromatographie**
Process for purification proenzyme of of the factor VII-activating protease, by ion-exchange chromatography
Procédé pour la purification du proenzmye de la protéase qui active le facteur VII, par chromatographie d'échange ionique

(30) Priorität: 06.08.1999 DE 19937219
(43) Veröffentlichungstag der Anmeldung: 07.02.2001
(73) Patentinhaber: CSL Behring GmbH, 35041 Marburg (DE)
(72) Erfinder: Römisch, Jürgen, Dr., 35041 Marburg (DE); Feussner, Annette, 35043 Marburg (DE); Stöhr, Hans-Arnold, 35093 Wetter (DE)

(56) Entgegenhaltungen:
- EP-A- 0 952 215
- EP-A- 1 074 615
- N-H CHOI-MIURA ET AL.PURIFICATION AND CHARACTERIZATION OF A NOVEL HYLAURONAN-BINDING PROTEIN (PHBP); IT HAS THREE EGF, A KRINGLE A: "nd a serine protease domain, similar to hepatocyte growth factor activator " JOURNAL OF BIOCHEMISTRY., Bd. 119, 1996, Seiten 1157-1165, XP000960750 JAPANESE BIOCHEMICAL SOCIETY, TOKYO., JP ISSN: 0021-924X
- RöMISCH J. ET AL: 'A protease isolated from human plasma activating Factor VII independent of tissue factor [XP 1036685]' BLOOD COAGULATION AND FIBRINOLYSIS Bd. 10, Nr. 8, Dezember 1999, Seiten 471 - 479
- HUNFELD A. ET AL: 'Detection of a novel plasma serine protease during purification of vitamin K-dependent coagulation factors [XP4260083]' FEBS LETTERS Bd. 456, Seiten 290 - 294

## Beschreibung

Gegenstand der Erfindung ist ein Verfahren zur Reindarstellung des Proenzyms der den Blutgerinnungsfaktor VII aktivierenden Protease sowie von pharmazeutischen Zubereitungen, die das genannte Protein enthalten.

Aus der deutschen Patentanmeldung 19 903 693.4 ist bereits eine Protease zur Aktivierung des Blutgerinnungsfaktors VII, ein Verfahren zu ihrer Gewinnung, zu ihrem Nachweis und zu ihrer Inaktivierung sowie Arzneizubereitungen bekannt, die diese Protease enthalten. Diese zuerst aus Plasma isolierte Protease kommt dort zusammen mit einer nicht-aktivierten Form vor, die im folgenden als "Proenzym" bezeichnet wird. Die Protease aktiviert den Blutgerinnungsfaktor VII und beschleunigt die Gerinnung, wie durch zahlreiche Experimente gezeigt werden konnte. Bei der weiteren Untersuchung der biologischen Eigenschaften dieses als Serin-Protease identifizierten Proteins stellte sich heraus, dass auch Einketten-Plasminogen-Aktivatoren wie die Prourokinase wirksam aktiviert werden. Außerdem wurde eine Inaktivierung der Faktoren V und VIII in vitro beobachtet. Zusätzlich zu den schon in der deutschen Patentanmeldung 19 903 693.4 beschriebenen sequenzierten Bereichen wurden N-terminale Sequenzierungen von Proteasefragmenten durchgeführt. Folgende Amino-säuresequenzen charakterisieren die FVII-aktivierende Protease: IYGGFK-STAGKHP; LLESLDPDXTPD; EFHEQSFRVEKI; SKFTXAXPXQFK; wobei X nicht identifiziert bedeutet. Die bisher aufgeklärten Sequenzen der genannten Protease zeigen, dass sie zu 100% mit Sequenzen der von Choi-Miura publizierten Protease übereinstimmen (Choi-Miura et al. J. Biochem. 1996; 119: 1157 bis 1165).

Die bisherigen Untersuchungen haben sich vor allem auf die Protease in ihrer aktivierten Form konzentriert. Die im Plasma als Proenzym vorliegende inaktive Form der Protease wurde erst vor kurzer Zeit anhand eines Protein-Bandenmusters in der SDS-PAGE nach Reduktion der Probe aufgefunden. Da bei der Aktivierung der Protease an einer für Serinproteasen typischen Stelle der Primärstruktur eine Spaltung und damit Aktivierung erfolgt, werden bei der Elektrophorese zwei oder mehr Banden sichtbar. Bei Reduktion der über Disulfidbrücken verbundenen Ketten werden die einzelnen Banden entsprechend ihrem niedrigerem Molekulargewicht erkennbar, wobei das Proenzym als große Einzelkette verbleibt. Dies wurde auch in komplexeren Lösungen nach Transfer der Proteine auf Membranen und anschließendes Western Blotting mit geeigneten Antikörpern deutlich.

Aus therapeutischen Gründen besteht nun ein Interesse daran, außer dem Gemisch der beiden genannten Proteine sowohl die Protease in ihrer aktivierten Form als auch das Proenzym zur Verfügung zu haben. Während man die aktivierte Protease zur raschen Aktivierung des Blutgerinnungsfaktors VII oder der Einketten-Plasminogenaktivatoren verwenden kann, um akute Krankheitsbilder zu beeinflussen, ist die Proenzymform der Protease vor allem für die mittel- bis längerfristige Prophylaxe oder Behandlung von angeborenen oder erworbenen Mangelzuständen oder auch zur Erhöhung des Plasmaspiegels über das physiologische Maß hinaus als bevorzugtes Mittel zu wählen. Dabei ist aber zu berücksichtigen, dass die Stabilisierung einer aktivierten Protease schwierig ist, da z.B. eine Eigendegradation stattfinden oder das Molekül aufgrund seiner strukturellen Gegebenheiten instabil sein kann. Bisherige Studien zeigten, dass die den Faktor VII aktivierende Protease nur unter besonderen Umständen in ihrer Proenzymform isoliert und stabilisiert werden kann.

Die bisher vorliegenden Untersuchungen haben gezeigt, dass die biologischen Aktivitäten dieser Protease durch Kalzium und/oder Heparin oder diesem verwandte Substanzen gesteigert werden können. Diese Eigenschaft wurde schon bisher genutzt, um die Protease an immobilisiertes Heparin zu adsorbieren und eine angereicherte Fraktion zu erhalten. Außerdem ist bereits bekannt, dass die Anionenaustauscher-Chromatographie ebenfalls zur Reinigung der Protease geeignet ist. Die Kombination beider Reinigungsschritte ist geeignet, die Protease in angereicherter Form zu gewinnen. Auch die Verwendung einer Aprotinin-Matrix kann zur Reindarstellung der aktivierten Protease verwendet werden.

Es wurde nun ein Verfahren zur Reindarstellung und gleichzeitigen Stabilisierung des Proenzyms der den Blutgerinnungsfaktor VII aktivierenden Protease gefunden, bei dem es aus biologischen oder den bei der gentechnologischen Herstellung anfallenden Flüssigkeiten durch
Anionen- und/oder Kationenaustauscher-Chromatographie bei einem pH-Wert zwischen 2,5 und 7,5 gewonnen wird.

Die Anionen- oder Kationenaustauscher-Chromatographie kann zusätzlich mit einer Affinitätschromatographie bei pH-Werten zwischen 2,5 und 9,0 kombiniert werden. Die genannten Verfahren zur Reinigung des Proenzyms können auch mit einer fraktionierten Fällung kombiniert werden.
Die Affinitätschromatographie wird unter Verwendung von
- Kalziumphosphat/Hydroxylapatit unter Verwendung von
- einer hydrophoben Matrix,
- einer Chelatmatrix,
- einer Matrix, die mit einem immobilisierten, gegen das zu isolierende Protein gerichteten, monoklonalen oder polyklonalen Antikörper oder seinen F(ab)- oder F(ab)₂-Fragmenten beschichtet ist, durchgeführt.

Eine besonders geeignete Methode zur Reindarstellung der Proenzyms der Protease ist die Anionen- und/oder Kationenaustauscher-Chromatographie. Der Einsatz dieser Methoden zur Reindarstellung der aktivierten Protease ist zwar schon früher vorgeschlagen worden, jedoch haben die bisher angewendeten Verfahrensbedingungen keine voll befriedigenden Ergebnisse erbracht. Das liegt vor allem daran, dass das Risiko der Aktivierung des Proenzyms bei der Kontaktierung mit den Oberflächen der Matrices sehr hoch ist. Es stellte sich deshalb die Aufgabe, ein Verfahren zu entwickeln, das die Darstellung der aktivierten Protease und des Proenzyms in reiner und stabiler Form ermöglicht.

Überraschenderweise zeigte sich nun, dass sehr niedrige pH-Werte, insbesondere pH-Werte zwischen 2,5 und 7,2 dem Proenzym nicht schaden und deshalb bei der Adsorption und Elution mit gutem Erfolg eingesetzt werden können. Hierdurch wird eine nahezu störungsfreie Handhabung des Proenzyms der den Faktor VII aktivierenden Protease ermöglicht, da die meisten anderen im Plasma zirkulierenden Proteasen im sauren Medium nicht oder nur sehr wenig aktiv sind und damit das Risiko einer proteolytischen Aktivierung minimiert wird. Auch die Gefahr des Eigenabbaus des Proenzyms wird auf diese Weise vermindert. Da bekanntlich extrem saure pH-Werte das Risiko einer Denaturierung beinhalten, wurde die Aktivität der aus einem stark sauren Medium gewonnenen Protease z.B. durch eine photometrische Bestimmung der bei der Einwirkung auf chromogene Substrate auftretenden Extinktion gemessen. Dabei konnte gezeigt werden, dass das Proenzym kurzfristig bis zu einem pH-Wert von 2,0 ohne Aktivitätsverlust gehandhabt werden kann. Bei einem pH-Wert von 2,5 bis etwa 7,2 kann das Proenzym über mehrere Monate gelagert werden, wobei die höchsten Stabilitäten bei einem pH-Wert von unter 6,5 beobachtet werden.

Überraschend war dabei der Befund, dass eine Anionen- und/oder Kationenaustauscher-Chromatographie bei den genannten niedrigen pH-Werten zur Reinigung des Proenzyms verwendet werden kann. Dies ist deshalb so bemerkenswert, weil dabei eine Adsorption an den Austauscher-Matrices bei pH-Werten möglich wird, die unterhalb des isoelektrischen Punktes oder des Proenzyms liegen.

Bisher gibt es noch keine wissenschaftliche Erklärung dafür, auf welchen Wechselwirkungen diese Adsorption beruht. Es gelingt jedoch durch diese Adsorption im sauren Medium eine Vielzahl von Verunreinigungen zu entfernen, die bei diesen pH-Werten nicht an die Matrices binden. Damit wird eine erhebliche Anreicherung der Protease auf der Matrix erzielt. Nach Waschen der Matrix kann die Protease und/oder das Proenzym durch eine Erhöhung der Ionenstärke eluiert werden.

Die Anionen- und/oder Kationenaustauscher-Chromatographie können zur Reindarstellung der vorstehend genannten Proteine mit einer Affinitätschromatographie und/oder einer fraktionierten Fällung kombiniert werden. Unabhängig davon, ob die vorstehend genannten Reinigungsverfahren einzeln oder in einer beliebigen Kombination eingesetzt werden, empfiehlt es sich, zusätzlich bei allen Verfahrensschritten Protease-Inhibitoren zuzusetzen, um eine Spaltung des Proenzyms in die aktivierte Protease zu blockieren. Als geeignete Proteinstabilisatoren werden dabei
- Lösungsvermittler, vorzugsweise Hydroxyprolin,
- Detergentien, vorzugsweise Tween^{®} oder Triton^{®},
- Proteine, vorzugsweise Albumin, Gelatine, Fibronektin und Vitronektin oder ähnlicher Proteine,
- Reduktionsmittel, vorzugsweise Dithiothreitol, Mercaptoethanol oder Cystein, und/oder
- Proteinase-Inhibitoren wie Aprotinin, α-2-Antiplasmin, C1-EsteraseInhibitor, Inter-α-Trypsin-Inhibitor, Antithrombinlll/Heparin oder synthetische Inhibitoren
zugesetzt.

Eine besonders gute Stabilisierung lässt sich bei der Reindarstellung der Protease und des Proenzyms erreichen, wenn als weitere Proteinstabilisatoren
- Komplexbildner zweiwertiger Ionen, vorzugsweise EGTA, EDTA oder Citrat, und/oder
- zweiwertige Ionen, vorzugsweise Kalziumionen, und/oder
- Aminosäuren, vorzugsweise Glutamat, Arginin, Lysin oder Glyzin, und/oder
- Zucker, vorzugsweise Glukose, Arabinose, Mannose oder Mannit, und/oder
- Alkohole, vorzugsweise Ethylenglykol oder Polyethylenglykol,
zugesetzt werden.

Die vorstehend genannten Verfahrensschritte können darüber hinaus auch mit einer fraktionierten Fällung des Proenzyms kombiniert werden, die aus ihrer Lösung durch Zusatz von
- Polyethylenglykol ab einer Konzentration von mindestens 10 Gew.-% oder
- Ammoniumsulfat ab einer Konzentration von mindestens 15 Gew.-% erfolgt.

Besonders bemerkenswert ist, dass bei den vorstehend beschriebenen Verfahren die Proenzymform der Protease in reiner Form erhalten werden kann. Es zeigte sich nämlich, dass die Ionenaustauscher-Chromatographie unter den genannten sauren Bedingungen mit einer Lösung, die vor allem das Proenzym enthielt, zu einem Eluat führte, das ausschließlich das Proenzym oder wenigstens in sehr stark angereicherten Ausmaß enthielt. Dabei lässt sich die Nativität des so erhaltenen Proenzyms mit Hilfe eines der Aktivitätsteste bestimmen, die in der deutschen Patentanmeldung 199 26 531.3 beschrieben sind, also z.B. durch die photometrische Bestimmung der bei Einwirkung auf chromogene Substrate auftretenden Extinktion oder durch die nach Reduktion der Probe auftretende Einkettenbildung, die durch SDS-PAGE/Westem Blotting nachgewiesen werden kann. Das zeigt, dass erfindungsgemäß die Präparation des Proenzyms in schneller und effizienter Weise mit hoher Ausbeute gelingt.

Bei Anwendung der vorstehend genannten Verfahrensschritte kann also das gereinigte Proenzym der den Faktor VII aktivierenden Protease erhalten werden. Ein besonders empfehlenswerter Weg zur Herstellung einer reinen aktivierten Protease besteht in der chromatographischen Trennung der den Faktor VII aktivierenden Protease von ihrem Proenzym mittels stufenweiser Elution, bei der auf dem Trägermaterial eine Substanz immobilisiert ist, die unterschiedlich starke Bindungen zur Protease einerseits und zum Proenzym andererseits aufweist. Damit können unterschiedliche Eluate gewonnen werden, die nur das Proenzym enthalten.

Zur Gewinnung des Proenzyms wird dabei auf der Matrix ein Inhibitor mit starke Affinität für die aktivierte Protease immobilisiert. Hierfür kommen vor allem Serinprotease-Inhibitoren, insbesondere der C1-Esterase-Inhibitor, das α-2-Antiplasmin, das Antithrombin III (unter Beimischung von Heparin zur Auftragslösung) oder niedermolekulare, hochaffine Inhibitoren, die auch synthetischer Natur sein können, in Frage. Im Säulendurchfluss befindet sich dann das an der Matrix nicht oder weniger fest gebundene Proenzym. Hingegen wird zur Reindarstellung der aktivierten Protease ein Inhibitor auf der Matrix immobilisiert, der nur ein schwaches inhibitorisches Potential aufweist und die aktivierte Protease reversibel bindet. In diesem Fall wird nach dem Auswaschen des Proenzyms die auf der Matrix gebundene aktive Protease eluiert und kann dann in reiner Form gewonnen werden. Für diese Verfahrensvariante eignet sich z.B. ein mit Aprotinin behandeltes Trägermaterial oder niedermolekulare, reversible Inhibitoren, die auch synthetischer Natur sein können.

Selbstverständlich können auch Antikörper oder deren Fragmente, die zwischen der aktivierten Protease und dem Proenzym unterscheiden können, für die Reindarstellung des genannten Proteines verwendet werden. Hierfür können vor allem monoklonale Antikörper eingesetzt werden, die "Neoepitope" nach Aktivierung der Protease erkennen können, also z.B. die Aktivierungs- oder Spaltstelle des Proteins.

Unter den erfindungsgemäß einzusetzenden affinitätschromatographischen Verfahren ist die Adsorption an Kalziumphosphat/Hydroxylapatit als eine einfache und schnelle Methode zur Anreicherung der Protease und/oder des Proenzyms hervorzuheben. Dabei wird die Lösung, die die Protease und das Proenzym enthält, bei einem pH-Wert zwischen 2,5 und 9,0, vorzugsweise zwischen 2,5 und 7,2 mit Kalziumphosphat versetzt. Nach anschließender Sedimentierung z.B. durch Zentrifugation oder durch Filtration wird das Sediment, ggf. nach ein- oder mehrfachem Resuspendieren in einer Pufferlösung unter Zugabe von bspw. 0,2 M Natriumcitrat eluiert. Das Proenzym befindet sich dann im Eluat.

Auch die Adsorption der Protease an hydrophoben Matrices oder an hydrophoben Liganden, die an ensprechende Matrices gekoppelt sind, kann erfindungsgemäß verwendet werden. Beispiele sind Phenyl- oder Octyl-Sepharosen^{®} oder ein an eine Matrix gekoppeltes Phenylalanin. Die Elution des gebundenen Proteins erfolgt in an sich bekannter Weise mit einer gepufferten Lösung geringer Ionenstärke, die Phenylalanin, Glycerin oder Ethylenglykol enthalten kann.

Da das Proenzym eine Wechselwirkung mit Kationen, vor allem mit Kalzium und Magnesiumionen eingeht, was durch eine Steigerung ihrer Aktivität in deren Gegenwart bestätigt wird, bietet sich zu deren Anreicherung aus entsprechenden Lösungen die Chromatographie mittels sogenannter "Chelat-Matrices" an. Chelatverbindungen mit Zink-, Kupfer- oder Nickelionen sind dabei besonders geeignet. Nach dem Waschen der mit der Protease beladenen Matrix kann zur Elution gebundener Proteine auch ein Imidazolpuffer, ggf. mit einem linearen Gradienten, eingesetzt werden.

Das erfindungsgemäße Verfahren kann auch einen affinitätschromatographischen Reinigungsschritt enthalten, bei dem als Trägermaterial eine Matrix eingesetzt wird, auf der gegen die genannten Proteine gerichtete, monoklonale oder polyklonale Antikörper oder ihre F(ab)- oder ihre F(ab)₂-Fragmente oder andere, zur reversiblen Bindung der genannten Proteine geeignete Substanzen immobilisiert sind.

Das nach dem erfindungsgemäßen Verfahren gewonnene Proenzym kann mit oder ohne den Zusatz zusätzlicher Stabilisatoren im sauren pH-Bereich bei pH-Werten von etwa 2,5 bis 7,2 ohne Aktivitätsverlust gelagert werden, während im alkalischen Bereich ein Zusatz von Stabilisatoren zwingend ist.

Therapeutisch kann das gennante Proenzym zur Unterstützung der Blutgerinnung bei Blutungsneigung, bei Mangel an Faktoren des endogenen Gerinnungsastes oder als FEIBA (= Factor VIII bypassing activity), aber auch zur endogenen und exogenen Aktivierung von Plasminogenaktivatoren wie der Prourokinase oder des Einketten-tPA verwendet werden. Diese Aktivität kann auch durch Anwendung der genannten Protease zur Prophylaxe oder Therapie von thromboembolischen Erkrankungen in Kombination mit Einketten- oder Zweiketten-Plasminogenaktivatoren oder Antikoagulantien eingesetzt werden. Krankheitsbilder, die mit thrombotischen Komplikationen vergesellschaftet sind, wie Herzinfarkt, Angina pectoris, Schlaganfall oder Beinvenenthrombosen können so erfolgreich behandelt werden.

Ein weiterer Gegenstand der Erfindung ist deshalb eine pharmazeutische Zubereitung, die eine zur Auflösung von fibrinhaltigen Thromben ausreichende Menge der Proenzym form der den Blutgerinnungsfaktor VII aktivierenden Protease enthält. Auch diese Zubereitung kann außerdem Einketten-Plasminogenaktivatoren und/oder Antikoagulantien enthalten.

Zweckmässigerweise wird der Zubereitung noch ein Proteinase-Stabilisator oder ein Reduktionsmittel wie Dithiothreitol, Mercaptoethanol oder Cystein zugesetzt, um das Risiko einer Polymerbildung während der Aufarbeitung oder bei der Lagerung zu reduzieren.

Auch bei Wundheilungsprozessen spielen fibrinolytische Prozesse eine Rolle. Dabei kann das genannte Proenzym intravenös oder lokal, subkutan, intradermal, intramuskulär oder bei Verletzungen und Wunden als Bestandteil eines Fibrinklebers oder auch topisch oder gebunden an eine geeignete Trägermatrix z.B. in Form eines Vliesses oder eines Pflasters erfolgen, wobei eine Kombination mit Wachstumsfaktoren zweckmässig sein kann. Im allgemeinen kommt eine derartige pharmazeutische Zubereitung in flüssiger oder lyophilisierter Form zur Anwendung, der an sich bekannte Proteinstabilisatoren zugesetzt werden können, also z.B. Komplexbildner, zweiwertige Kationen wie Kalzium, Aminosäuren wie Glutamat, Arginin, Lysin oder Glyzin und/oder Zucker wie Glukose, Arabinose, Mannose oder Mannitol.

Außerdem kann das Proenzym auch zur Beschichtung von in den Organismus zu implantierenden, aus Kunststoffen oder Metallen bestehenden Gegenständen, wie künstlichen Herzklappen, Blutgefässen, aber auch zur Blutentnahme oder zur künstlichen Ernährung eingeführten Kanülen eingesetzt werden.

Die Erfindung wird durch die folgenden Beispiele erläutert:

### Beispiel 1 (Zum Vergleich)

### Reindarstellung mittels immobilisierter monoklonaler Antikörper

Monoklonale Antikörper, die gegen die den Faktor VII aktivierende Protease gerichtet sind, wurden an BrCN-Sepharose^{®} gekoppelt. 30 ml dieser mAb-Matrix wurden in eine Säule gefüllt und das Harz mit 50 mM Natriumcitrat, 0,1 M Natriumchlorid NaCl, 0,1 M Arginin x HCl, pH 6,0, equilibriert.

100 ml Citratplasma wurden über die Säule gepumpt und die Matrix dann mit 50 mM Natriumcitrat, 1 M NaCl, 0,1 M Arginin x HCI, pH 6,0, gewaschen. Danach wurde die Säule mit dem Equilibrierungspuffer erneut gewaschen, woran sich die Elution mit 0,1 Glyzin, pH 2,5, anschloss. Das Eluat (ca. 30 ml) wurde in einer Vorlage von 3 ml einer 200 mM Natriumcitratlösung, pH 5,5 unter Rühren gesammelt und danach auf einen pH-Wert von 4,5 eingestellt.

Die Eluatlösung wurde zur weiteren Analyse verwendet. Es wurde eine SDS-PAGE mit anschließendem Transfer auf eine PVDF-Membran und eine Detektion der Faktor VII-Aktivator-Bande mit der nicht reduzierten und mit der reduzierten Probe durchgeführt. Aktivitätstests der so gewonnenen Proteine wurden entsprechend dem in der deutschen Patentanmeldung 199 26 531.3 beschriebenen Verfahren, nämlich der Aktivierung von Prourokinase und Faktor VII, mit anschließender Detektion von Urokinase oder dem aktivierten Faktor VII durchgeführt. Die in diesem System getesteten Proteasemengen, bestimmt als Proteaseantigen, entsprechen der erwarteten theoretischen Aktivität, wodurch die Aktivität der isolierten Protease oder des Proenzyms hinsichtlich der biologischen Aktivität gezeigt wurde.

### Beispiel 2

### Anionenaustauscher-Chromatographie

Eine die Proenzymform der Faktor VII-aktivierenden Protease enthaltende Lösung, die noch Verunreinigungen durch andere Proteine enthielt, wurde in einer Pufferlösung aus 20 mM Na-Acetat, 0,1 M Glyzin, pH 4,5, auf eine Mono Q-Sepharose gepumpt und anschließend mit dem o.g. Puffer gewaschen. Die Durchlauf-Fraktion wurde aufgefangen. Die Elution gebundener Proteine erfolgte mit 20 mM Na-Acetat, 2 M NaCl, pH 4,5. Das Eluat wurde in einen Puffer aus 5 mM Na-Citrat, 50 mM NaCl, pH 6,0, verdünnt und in den in Beispiel 1 genannten Testsystemen untersucht. Aliquots wurden bei 4 bis 8°C gelagert bzw. bei -20°C eingefroren.

Nach Lagerung der Eluatlösung bei 6°C für mehrere Tage wurden die Tests wiederholt, wobei die Verdünnungen der (aufgetauten) Proben jeweils kurz vor dem Test durchgeführt wurden.

SDS-PAGEs/Western Blots bestätigten, dass die Protease in ihrer Proenzymform isoliert worden war. Nach SDS-PAGE und Anfärbung von Proteinen mittels Coomassie Blue waren in der Ausgangslösung (vor Chromatographie) neben der Protease eine Reihe von verunreinigenden Proteinen sichtbar, die ebenfalls in den Durchlauffraktionen zu finden waren. Die Protease stellte sich als eine Bande entsprechend der Proenzymform (also auch nach Reduktion) rein dar. Die Aktivitätstests (siehe Beispiel 1) bestätigten die Nativität der Protease im Sinne der Beibehaltung der biologischen Aktivitäten.

## Patentansprüche

1. Verfahren zur Darstellung des Proenzyms der den Blutgerinnungsfaktor VII aktivierenden Protease in reiner und stabiler Form, **dadurch gekennzeichnet, dass** das Proenzym der den Blutgerinnungsfaktor VII aktivierenden Protease aus biologischen oder aus bei der gentechnologischen Herstellung anfallenden Flüssigkeiten durch Anionen- und/oder Kationenaustauscher Chromatographie bei einem pH-Wert zwischen 2,5 und 7,2 gewonnen wird.

2. Verfahren nach Anspruch 1, **dadurch gekennzeichnet, dass** es mit einer Affinitätschromatographie bei einem pH-Wert zwischen 2,5 und 9,0 kombiniert wird.

3. Verfahren nach Anspruch 2, wobei eine Affinitätschromatographie unter Verwendung von
• Kalziumphosphat/Hydroxylapatit oder
• einer hydrophoben Matrix oder
• einer Chelatmatrix oder
• einer Matrix, die mit einem immobilisierten, gegen das Proenzym der den Blutgerinnungsfaktor VII aktivierenden gerichteten monoklonalen oder polyklonalen Antikörper oder seinen F(ab)- oder F(ab)₂-Fragmenten beschichtet ist,
durchgeführt wird.

4. Verfahren nach einem der Ansprüche 1 bis 3, **dadurch gekennzeichnet, dass** es mit einer fraktionierten Fällung des Proenzyms kombiniert wird.

5. Verfahren nach Anspruch 4, **dadurch gekennzeichnet, dass** die fraktionierte Fällung des Proenzyms aus ihrer Lösung durch Zusatz von
- Polyethylenglykol ab einer Konzentration von mindestens 10 Gew.% oder
- Ammoniumsulfat ab einer Konzentration von mindestens 15 Gew.% erfolgt.

6. Verfahren nach einem der Ansprüche 1 bis 5, **dadurch gekennzeichnet, dass** die Reindarstellung in Gegenwart von einem oder mehreren Proteinstabilisatoren erfolgt, die ausgewählt werden aus
- Lösungsvermittlern, vorzugsweise Hydroxyprolin,und
- Detergentien, vorzugsweise Tween^{®} und Triton^{®}, und
- Proteinen, vorzugsweise Albumin, Gelatine, Fibronektin, Vibronektin oder ähnlichen Proteinen, und
- Reduktionsmitteln, vorzugsweise Dithiothreitol, Mercaptoethanol oder Cystein, und
- Proteinase-Inhibitoren wie Aprotinin, α-2-Antiplasmin, C1-EsteraseInhibitor, dem Inter-α-Trypsin-Inhibitor, dem AntithrombinIII/ Heparin und synthetischen Inhibitoren.

7. Verfahren nach Anspruch 6, **dadurch gekennzeichnet, dass** bei der Reindarstellung als weitere Proteinstabilisatoren
- Komplexbildner zweiwertiger Ionen, vorzugsweise EGTA, EDTA oder Citrat, und/oder
- zweiwertige Ionen, vorzugsweise Kalziumionen, und/oder
- Aminosäuren, vorzugsweise Glutamat, Arginin, Lysin oder Glyzin, und/oder
- Zucker, vorzugsweise Glukose, Arabinose, Mannose oder Mannit, und/oder
- Alkohole, vorzugsweise Ethylenglykol oder Polyethylenglykol, eingesetzt werden.

8. Pharmazeutische Zubereitung, **dadurch gekennzeichnet, dass** sie das Proenzym der den Blutgerinnungsfaktor VII aktivierenden Protease in reiner und stabiler Form zusammen mit einem oder mehreren Proteinstabilisatoren ausgewählt aus der Gruppe umfassend :
- Lösungsvermittler, vorzugsweise Hydroxyprolin, und
- Detergentien, vorzugsweise Tween^{®} und Triton^{®}, und
- Proteine, vorzugsweise Albumin, Gelatine, Fibronektin, Vibronektin oder ähnliche Proteine, und
- Reduktionsmittel, vorzugsweise Dithiothreitol, Mercaptoethanol oder Cystein, und
- Proteinase-Inhibitoren wie Aprotinin, α-2-Antiplasmin, C1-EsteraseInhibitor, Inter-α-Trypsin-Inhibitor, AntithrombinIII/ Heparin und synthetische Inhibitoren,
enthält,
zur Unterstützung der Blutgerinnung bei Blutungsneigung, bei Mangel an Faktoren des endogenen Gerinnungsweges, als FEIBA, zur Prophylaxe und/oder Therapie von mit thrombotischen Komplikationen vergesellschafteten Krankheitsbildern, bei angeborenen oder erworbenen Mangelzuständen an der Protease oder ihrem Proenzym, zur Unterstützung der Wundheilung allein oder als Bestandteil eines Fibrinklebers, eines Vlieses oder in Kombination mit Wachstumsfaktoren, zur subkutanen oder intramuskulären, intravenösen oder topischen Behandlung.

9. Pharmazeutische Zubereitung gemäß Anspruch 8, **dadurch gekennzeichnet, dass** zusätzlich ein oder mehrere Proteinstabilisatoren ausgewählt aus der Gruppe umfassend:
Komplexbildner zweiwertiger Ionen, vorzugsweise EGTA, EDTA oder Citrat, und
- zweiwertige Ionen, vorzugsweise Kalziumionen, und
- Aminosäuren, vorzugsweise Glutamat, Arginin, Lysin oder Glyzin, und
- Zucker, vorzugsweise Glukose, Arabinose, Mannose oder Mannit, und
- Alkohole, vorzugsweise Ethylenglykol oder Polyethylenglykol,
eingesetzt werden.

10. Verwendung einer pharmazeutischen Zubereitung gemäß Anspruch 8 oder 9 zur Beschichtung von Oberflächen von in den Organismus zu implantierenden, aus Kunststoff oder Metallen bestehenden Gegenständen, wie künstlichen Herzklappen, Blutgefäßen oder auch zur Blutentnahme oder zur künstlichen Ernährung eingeführten Kanülen.

11. Reagenz enthaltend das Proenzym der den Blutgerinnungsfaktor VII aktivierenden Protease in reiner und stabiler Form zusammen mit einem oder mehreren Proteinstabilisatoren ausgewählt aus der Gruppe umfassend:
- Lösungsvermittler, vorzugsweise Hydroxyprolin, und
- Detergentien, vorzugsweise Tween^{®} und Triton^{®}, und
- Proteine, vorzugsweise Albumin, Gelatine, Fibronektin, Vibronektin oder ähnliche Proteinen, und
- Reduktionsmittel, vorzugsweise Dithiothreitol, Mercaptoethanol oder Cystein, und
- Proteinase-inhibitoren wie Aprotinin, α-2-Antiplasmin, C1-EsteraseInhibitor, Inter-α-Trypsin-Inhibitor, AntithrombinIII/ Heparin und synthetische Inhibitoren.
zur Verwendung in biologischen Testsystemen und zum Antigennachweis.

12. Reagenz gemäß Anspruch 12, **dadurch gekennzeichnet, dass** zusätzlich ein oder mehrere Proteinstabilisatoren ausgewählt aus der Gruppe umfassend:
Komplexbildner zweiwertiger Ionen, vorzugsweise EGTA, EDTA oder Citrat, und
- zweiwertige Ionen, vorzugsweise Kalziumionen, und
- Aminosäuren, vorzugsweise Glutamat, Arginin, Lysin oder Glyzin, und
- Zucker, vorzugsweise Glukose, Arabinose, Mannose oder Mannit, und
- Alkohole, vorzugsweise Ethylenglykol oder Polyethylenglykol,
eingesetzt werden.

## Claims

1. Process for the preparation of the proenzyme of the protease activating blood clotting factor VII in pure and stable form, **characterized in that** the proenzyme of the protease activating blood clotting factor VII is obtained from biological fluids or those obtained in the case of preparation by genetic engineering by anion- and/or cation-exchange chromatography at a pH of between 2.5 and 7.2.

2. Process according to Claim 1, **characterized in that** it is combined with an affinity chromatography at a pH of between 2.5 and 9.0.

3. Process according to Claim 2, wherein an affinity-chromatography separation process is carried out using
• calcium phosphate/hydroxyapatite or
• a hydrophobic matrix or
• a chelate matrix or
• a matrix which is coated with an immobilized monoclonal or polyclonal antibody directed against the proenzyme of the protease activating blood clotting factor VII, or its F(ab) or F(ab)₂ fragments.

4. Process according to any of Claims 1 to 3, **characterized in that** it is combined with a fractional precipitation of the proenzyme.

5. Process according to Claim 4, **characterized in that** the fractional precipitation of the proenzyme from its solution is carried out by addition of
- polyethylene glycol from a concentration of at least 10% by weight or
- ammonium sulfate from a concentration of at least 15% by weight.

6. Process according to any of Claims 1 to 5, **characterized in that** the preparation in pure form is carried out in the presence of one or more protein stabilizers which are selected from the groups consisting of
- solubilizers, preferably hydroxyproline, and
- detergents, preferably Tween^{®} and Triton^{®}, and
- proteins, preferably albumin, gelatin, fibronectin, vitronectin or similar proteins, and
- reductants, preferably dithiothreitol, mercaptoethanol or cystienee, and
- proteinase inhibitors such as aprotinin, α-2-antiplasmin, C1-esterase inhibitor, the inter-α-trypsin inhibitor, the antithrombin III/heparin and synthetic inhibitors.

7. Process according to Claim 6, **characterized in that**, in the preparation in pure form, further protein stabilizers which are employed are
- complexing agents of divalent ions, preferably EGTA, EDTA or citrate and/or
- divalent ions, preferably calcium ions, and/or
- amino acids, preferably glutamate, arginine, lysine or glycine, and/or
- sugars, preferably glucose, arabinose, mannose or mannitol, and/or
- alcohols, preferably ethylene glycol or polyethylene glycol.

8. Pharmaceutical preparation, **characterized in that** it comprises the proenzyme of the protease activating blood clotting factor VII in pure and stable form together with one or more protein stabilizers selected from the group comprising:
- solubilizers, preferably hydroxyproline, and
- detergents, preferably Tween® and Triton®, and
- proteins, preferably albumin, gelatin, fibronectin, vitronectin or similar proteins, and
- reductants, preferably dithiothreitol, mercaptoethanol or cystienee, and
- proteinase inhibitors such as aprotinin, α-2-antiplasmin, C1-esterase inhibitor, inter-α-trypsin inhibitor, antithrombin III/ heparin and synthetic inhibitors,
for assisting blood clotting in the case of a tendency to bleeding, in the case of absence of factors of the endogenous clotting pathway, as FEIBA or for the prophylaxis and/or therapy of syndromes associated with thrombotic complications, in inherited or acquired deficiency states of the protease or its proenzyme, for assisting wound healing alone or as a constituent of a fibrin adhesive, a web or in combination with growth factors, for subcutaneous or intramuscular, intravenous or topical treatment.

9. Pharmaceutical preparation according to Claim 8, **characterized in that**, in addition, one or more protein stabilizers are employed which are selected from the group comprising:
- complexing agents of divalent ions, preferably EGTA, EDTA or citrate and
- divalent ions, preferably calcium ions, and
- amino acids, preferably glutamate, arginine, lysine or glycine, and
- sugars, preferably glucose, arabinose, mannose or mannitol, and
- alcohols, preferably ethylene glycol or polyethylene glycol.

10. Use of a pharmaceutical preparation according to Claim 8 or 9 for the coating of surfaces of articles, consisting of plastic or metals, to be implanted in the body, such as synthetic heart valves, blood vessels or cannulas inserted for taking blood or for artificial feeding.

11. Reagent comprising the proenzyme of the protease activating blood clotting factor VII in pure and stable form together with one or more protein stabilizers selected from the group comprising:
- solubilizers, preferably hydroxyproline, and
- detergents, preferably Tween^{®} and Triton^{®}, and
- proteins, preferably albumin, gelatin, fibronectin, vitronectin or similar proteins, and
- reductants, preferably dithiothreitol, mercaptoethanol or cystienee, and
- proteinase inhibitors such as aprotinin, α-2-antiplasmin, C1-esterase inhibitor, inter-α-trypsin inhibitor, antithrombin III/ heparin and synthetic inhibitors for use in biological test systems and for antigen detection.

12. Reagent according to Claim 12, charcterized in that, in addition, one or more protein stabilizers are employed which are selected from the group comprising:
- complexing agents of divalent ions, preferably EGTA, EDTA or citrate and
- divalent ions, preferably calcium ions, and
- amino acids, preferably glutamate, arginine, lysine or glycine, and
- sugars, preferably glucose, arabinose, mannose or mannitol, and
- alcohols, preferably ethylene glycol or polyethylene glycol.

## Revendications

1. Procédé pour préparer la proenzyme de la protéase activatrice du facteur VII de coagulation sanguine sous forme pure et stable, **caractérisé en ce que** la proenzyme de la protéase activatrice du facteur VII de coagulation sanguine est obtenue à partir de fluides biologiques, ou de fluides issus de la production par génie génétique, via une chromatographie échangeuse d'anions et/ou de cations à une valeur de pH comprise entre les valeurs 2,5 et 7,2.

2. Procédé selon la revendication 1, **caractérisé en ce que** celui-ci est combiné avec une chromatographie d'affinité à une valeur de pH comprise entre les valeurs 2,5 et 9,0.

3. Procédé selon la revendication 2, dans lequel on réalise une chromatographie d'affinité en utilisant :
- un système phosphate de calcium/hydroxyapatite, ou
- une matrice hydrophobe, ou
- une matrice de chélation, ou
- une matrice qui est revêtue d'un anticorps monoclonal ou polyclonal immobilisé, dirigé contre la proenzyme de la protéase activatrice du facteur VII de coagulation sanguine, ou qui est revêtue de ses fragments F(ab) ou F(ab)₂.

4. Procédé selon l'une quelconque des revendications 1 à 3, **caractérisé en ce que** l'on combine ce dernier avec une méthode de précipitation fractionnée de la proenzyme.

5. Procédé selon la revendication 4, **caractérisé en ce que** la précipitation fractionnée de la proenzyme est réalisée à partir de sa solution en ajoutant :
- du polyéthylène glycol en concentration à partir d'au moins 10 % en poids, ou
- du sulfate d'ammonium en concentration à partir d'au moins 15 % en poids.

6. Procédé selon l'une quelconque des revendications 1 à 5, **caractérisé en ce que** la préparation sous forme pure se fait en présence d'un ou de plusieurs stabilisateurs de protéine, que l'on choisit parmi :
- des solubilisants, de préférence, l'hydroxyproline,
- des détergents, de préférence, les produits Tween® et Triton®,
- des protéines, de préférence, l'albumine, la gélatine, la fibronectine, la vibronectine ou des protéines similaires,
- des agents réducteurs, de préférence, le dithiothréitol, le mercapto-éthanol ou la cystéine, et
- des inhibiteurs de protéases, tels que l'aprotinine, la α-2-antiplasmine, l'inhibiteur de l'estérase C1, l'inhibiteur de l'inter-α-trypsine, le système antithrombine III/héparine et les inhibiteurs synthétiques.

7. Procédé selon la revendication 6, **caractérisé en ce que** l'on utilise, lors de la préparation sous forme pure, comme autres stabilisateurs de protéine :
- des agents de complexation d'ions divalents, de préférence, l'EGTA, l'EDTA ou le citrate, et/ou
- des ions divalents, de préférence, les ions calcium, et/ou
- des acides aminés, de préférence, le glutamate, l'arginine, la lysine ou la glycine, et/ou
- des sucres, de préférence, le glucose, l'arabinose, le mannose ou le mannitol, et/ou
- des alcools, de préférence, l'éthylène glycol ou le polyéthylène glycol.

8. Préparation pharmaceutique, **caractérisée en ce qu'**elle contient la proenzyme de la protéase activatrice du facteur VII de coagulation sanguine sous une forme pure et stable, conjointement avec un ou plusieurs stabilisateurs de protéine que l'on choisit dans le groupe comprenant :
- des solubilisants, de préférence, l'hydroxyproline,
- des détergents, de préférence, les produits Tween^{®} et Triton^{®},
- des protéines, de préférence, l'albumine, la gélatine, la fibronectine, la vibronectine ou des protéines similaires,
- des agents réducteurs, de préférence, le dithiothréitol, le mercapto-éthanol ou la cystéine, et
- des inhibiteurs de protéases, tels que l'aprotinine, la α-2-antiplasmine, l'inhibiteur de l'estérase C1, l'inhibiteur de l'inter-α-trypsine, le système antithrombine III/héparine et des inhibiteurs synthétiques,
dans le but de soutenir la coagulation sanguine dans les cas de tendance hémorragique, de carence en facteurs de la voie de coagulation endogène, sous la forme FEIBA, pour la prophylaxie et/ou la thérapie de symptômes cliniques associés à des complications thrombotiques, dans le cas d'états de déficiences, innées ou acquises, en protéase ou de sa proenzyme, dans le but de soutenir la cicatrisation, sous forme isolée ou comme élément d'une colle de fibrine, d'un voile ou en combinaison avec des facteurs de croissance, pour effectuer un traitement par voie sous-cutanée ou intramusculaire, intraveineuse ou topique.

9. Préparation pharmaceutique selon la revendication 8, **caractérisée en ce que** l'on utilise, en plus, un ou plusieurs stabilisateurs de protéine, que l'on choisit dans le groupe comprenant :
- les agents de complexation d'ions divalents, de préférence, l'EGTA, l'EDTA ou le citrate, et
- les ions divalents, de préférence, les ions calcium,
- les acides aminés, de préférence, le glutamate, l'arginine, la lysine ou la glycine,
- les sucres, de préférence, le glucose, l'arabinose, le mannose ou le mannitol, et
- les alcools, de préférence, l'éthylène glycol ou le polyéthylène glycol.

10. Utilisation d'une préparation pharmaceutique selon la revendication 8 ou 9, pour revêtir les surfaces d'objets que l'on souhaite implanter dans l'organisme et qui sont constitués d'un matériau synthétique ou de métaux, par exemple, les valvules cardiaques, les vaisseaux sanguins artificiels ou, également, les canules introduites pour réaliser un prélèvement sanguin ou une alimentation artificielle.

11. Réactif contenant la proenzyme de la protéase activatrice du facteur VII de coagulation sanguine sous forme pure et stable, conjointement avec un ou plusieurs stabilisateurs de protéine choisis dans le groupe comprenant :
- les solubilisants, de préférence, l'hydroxyproline,
- les détergents, de préférence, les produits Tween® et Triton®,
- les protéines, de préférence, l'albumine, la gélatine, la fibronectine, la vibronectine ou des protéines similaires, et
- les agents réducteurs, de préférence, le dithiothréitol, le mercapto-éthanol ou la cystéine, et
- les inhibiteurs de protéases, tels que l'aprotinine, la α-2-antiplasmine, l'inhibiteur de l'estérase C1, l'inhibiteur de l'inter-α-trypsine, le système antithrombine III/héparine et les inhibiteurs synthétiques, pour une utilisation dans les systèmes d'essais biologiques et pour la détection d'antigènes.

12. Réactif selon la revendication 12, **caractérisé en ce que** l'on utilise, en outre, un ou plusieurs stabilisateurs de protéine, que l'on choisit dans le groupe comprenant :
- les agents de complexation d'ions divalents, de préférence, l'EGTA, l'EDTA ou le citrate, et
- les ions divalents, de préférence, les ions de calcium, et
- les acides aminés, de préférence, le glutamate, l'arginine, la lysine ou la glycine, et
- les sucres, de préférence, le glucose, l'arabinose, le mannose ou le mannitol, et
- les alcools, de préférence, l'éthylène glycol ou le polyéthylène glycol.
